# EUROPEAN PATENT APPLICATION

(11) **EP 3 138 579 A1**
(43) Date of publication of application: **08.03.2017**
(21) Application number: 15183983.4
(22) Date of filing: 05.09.2015
(51) Int. Cl.: A61K 39/29, A61K 39/36, C12N 15/62, A61P 31/20

(54) **FUSION PROTEIN FOR USE IN THE TREATMENT OF A HEPATITIS B VIRUS INFECTION**

(71) Applicant: BIOMAY AG, 1090 Wien (AT)
(72) Inventor: Valenta, Rudolf, 2604 Theresienfeld (AT); Cornelius, Carolin, 1090 Vienna (AT)
(74) Representative: KLIMENT & HENHAPEL

(57) **Abstract**

The present invention relates to a fusion protein for use in the treatment and/or prevention of a hepatitis B virus infection comprising at least one hepatitis B PreS polypeptide or fragment thereof fused to at least one peptide consisting of an amino acid sequence having at least 80% identity to a sequence selected from the group consisting of SEQ ID No. 1, SEQ ID No. 2, SEQ ID No. 3 and SEQ ID No. 4.

SEQUENCE LISTING

<110>  Biomay AG

<120>  Fusion protein

<130>  47640

<160>  24

<170>  PatentIn version 3.5

<210>  1
<211>  30
<212>  PRT
<213>  Artificial Sequence

<220>
<223>  Artificial peptide

<400>  1

Glu Ala Ala Phe Asn Asp Ala Ile Lys Ala Ser Thr Gly Gly Ala Tyr
1               5                   10                  15

Glu Ser Tyr Lys Phe Ile Pro Ala Leu Glu Ala Ala Val Lys
            20                  25                  30

<210>  2
<211>  37
<212>  PRT
<213>  Artificial Sequence

<220>
<223>  Artificial peptide

<400>  2

Ala Glu Glu Val Lys Val Ile Pro Ala Gly Glu Leu Gln Val Ile Glu
1               5                   10                  15

Lys Val Asp Ala Ala Phe Lys Val Ala Ala Thr Ala Ala Asn Ala Ala
            20                  25                  30

Pro Ala Asn Asp Lys
        35

<210>  3
<211>  33
<212>  PRT
<213>  Artificial Sequence

<220>
<223>  Artificial peptide

<400>  3

Ala Asp Leu Gly Tyr Gly Pro Ala Thr Pro Ala Ala Pro Ala Ala Gly

1               5                   10                  15

Tyr Thr Pro Ala Thr Pro Ala Ala Pro Ala Glu Ala Ala Pro Ala Gly
            20                  25                  30

Lys

<210>  4
<211>  33
<212>  PRT
<213>  Artificial Sequence

<220>
<223>  Artificial peptide

<400>  4

Ala Thr Thr Glu Glu Gln Lys Leu Ile Glu Lys Ile Asn Ala Gly Phe
1               5                   10                  15

Lys Ala Ala Leu Ala Ala Ala Ala Gly Val Gln Pro Ala Asp Lys Tyr
            20                  25                  30

Arg

<210>  5
<211>  173
<212>  PRT
<213>  Hepatitis B virus

<400>  5

Gly Gly Trp Ser Ser Lys Pro Arg Lys Gly Met Gly Thr Asn Leu Ser
1               5                   10                  15

Val Pro Asn Pro Leu Gly Phe Phe Pro Asp His Gln Leu Asp Pro Ala
            20                  25                  30

Phe Gly Ala Asn Ser Asn Asn Pro Asp Trp Asp Phe Asn Pro Ile Lys
        35                  40                  45

Asp His Trp Pro Ala Ala Asn Gln Val Gly Val Gly Ala Phe Gly Pro
    50                  55                  60

Gly Leu Thr Pro Pro His Gly Gly Ile Leu Gly Trp Ser Pro Gln Ala
65                  70                  75                  80

Gln Gly Ile Leu Thr Thr Val Ser Thr Ile Pro Pro Pro Ala Ser Thr
                85                  90                  95

Asn Arg Gln Ser Gly Arg Gln Pro Thr Pro Ile Ser Pro Pro Leu Arg
            100                 105                 110

Asp Ser His Pro Gln Ala Met Gln Trp Asn Ser Thr Ala Phe His Gln
        115                 120                 125

Ala Leu Gln Asp Pro Arg Val Arg Gly Leu Tyr Phe Pro Ala Gly Gly
    130                 135                 140

Ser Ser Ser Gly Thr Val Asn Pro Ala Pro Asn Ile Ala Ser His Ile
145                 150                 155                 160

Ser Ser Ile Ser Ala Arg Thr Gly Asp Pro Val Thr Asn
                165                 170

<210> 6
<211> 307
<212> PRT
<213> Artificial Sequence

<220>
<223> PreSF3

<400> 6

Met Glu Ala Ala Phe Asn Asp Ala Ile Lys Ala Ser Thr Gly Gly Ala
1               5                   10                  15

Tyr Glu Ser Tyr Lys Phe Ile Pro Ala Leu Glu Ala Ala Val Lys Ala
            20                  25                  30

Glu Glu Val Lys Val Ile Pro Ala Gly Glu Leu Gln Val Ile Glu Lys
        35                  40                  45

Val Asp Ala Ala Phe Lys Val Ala Ala Thr Ala Ala Asn Ala Ala Pro
    50                  55                  60

Ala Asn Asp Lys Gly Gly Trp Ser Ser Lys Pro Arg Lys Gly Met Gly
65                  70                  75                  80

Thr Asn Leu Ser Val Pro Asn Pro Leu Gly Phe Phe Pro Asp His Gln
                85                  90                  95

Leu Asp Pro Ala Phe Gly Ala Asn Ser Asn Asn Pro Asp Trp Asp Phe
            100                 105                 110

Asn Pro Ile Lys Asp His Trp Pro Ala Ala Asn Gln Val Gly Val Gly
        115                 120                 125

Ala Phe Gly Pro Gly Leu Thr Pro Pro His Gly Gly Ile Leu Gly Trp
    130                 135                 140

Ser Pro Gln Ala Gln Gly Ile Leu Thr Thr Val Ser Thr Ile Pro Pro
145                 150                 155                 160

Pro Ala Ser Thr Asn Arg Gln Ser Gly Arg Gln Pro Thr Pro Ile Ser
                165                 170                 175

Pro Pro Leu Arg Asp Ser His Pro Gln Ala Met Gln Trp Asn Ser Thr
            180                 185                 190

Ala Phe His Gln Ala Leu Gln Asp Pro Arg Val Arg Gly Leu Tyr Phe
        195                 200                 205

Pro Ala Gly Gly Ser Ser Ser Gly Thr Val Asn Pro Ala Pro Asn Ile
    210                 215                 220

Ala Ser His Ile Ser Ser Ile Ser Ala Arg Thr Gly Asp Pro Val Thr
225                 230                 235                 240

Asn Ala Asp Leu Gly Tyr Gly Pro Ala Thr Pro Ala Ala Pro Ala Ala
                245                 250                 255

Gly Tyr Thr Pro Ala Thr Pro Ala Ala Pro Ala Glu Ala Ala Pro Ala
            260                 265                 270

Gly Lys Ala Thr Thr Glu Glu Gln Lys Leu Ile Glu Lys Ile Asn Ala
        275                 280                 285

Gly Phe Lys Ala Ala Leu Ala Ala Ala Ala Gly Val Gln Pro Ala Asp
    290                 295                 300

Lys Tyr Arg
305

<210>  7
<211>  173
<212>  PRT
<213>  Hepatitis B virus

<400>  7

Gly Gly Trp Ser Ser Lys Pro Arg Lys Gly Met Gly Thr Asn Leu Ser
1               5                   10                  15

Val Pro Asn Pro Leu Gly Phe Phe Pro Asp His Gln Leu Asp Pro Ala
            20                  25                  30

Phe Lys Ala Asn Ser Asp Asn Pro Asp Trp Asp Leu Asn Pro His Lys
        35                  40                  45

Asp Asn Trp Pro Asp Ser Asn Lys Val Gly Val Gly Ala Phe Gly Leu
    50                  55                  60

Gly Phe Thr Pro Pro His Gly Gly Leu Leu Gly Trp Ser Pro Gln Ala
65                  70                  75                  80

Gln Gly Ile Leu Thr Thr Val Pro Ala Ala Pro Pro Pro Ala Ser Thr
                85                  90                  95

Asn Arg Gln Val Ala Arg Pro Pro Thr Pro Leu Ser Pro Pro Leu Arg
            100                 105                 110

Asp Thr His Pro Gln Ala Met Gln Trp Asn Ser Thr Thr Phe His Gln
        115                 120                 125

Thr Leu Gln Asp Pro Arg Val Arg Ala Leu Tyr Phe Pro Ala Gly Gly
    130                 135                 140

Ser Ser Ser Gly Thr Val Asn Pro Val Gln Asn Thr Ala Ser Ser Ile
145                 150                 155                 160

Ser Ser Ile Leu Ser Lys Thr Gly Asp Pro Val Pro Asn
                165                 170


<210>  8
<211>  173
<212>  PRT
<213>  Hepatitis B virus

<400>  8

Gly Gly Tyr Ser Ser Lys Pro Arg Lys Gly Met Gly Thr Asn Leu Ser
1               5                   10                  15

Val Pro Asn Pro Leu Gly Phe Leu Pro Asp His Gln Leu Asp Pro Ala
            20                  25                  30

Phe Gly Ala Asn Ser Asn Asn Pro Asp Trp Asp Phe Asn Pro Asn Lys
        35                  40                  45

Asp Pro Trp Pro Glu Ala Trp Gln Val Gly Val Gly Ala Phe Gly Pro
    50                  55                  60

Gly Phe Thr Pro Pro His Gly Gly Leu Leu Gly Trp Ser Pro Gln Ala
65                  70                  75                  80

Gln Gly Ile Leu Thr Pro Val Pro Ala Ala Pro Pro Pro Ala Ser Thr
                85                  90                  95

Asn Arg Gln Ser Gly Arg Gln Pro Thr Pro Ile Ser Pro Pro Leu Arg
            100                 105                 110

Asp Ser His Pro Gln Ala Met Gln Trp Asn Ser Thr Thr Phe His Gln
        115                 120                 125

Ala Leu Leu Asp Pro Arg Val Arg Gly Leu Tyr Phe Pro Ala Gly Gly
    130                 135                 140

Ser Ser Ser Gly Thr Thr Asn Pro Val Pro Thr Thr Ala Ser Pro Ile
145                 150                 155                 160

Ser Ser Ile Phe Ser Arg Thr Gly Asp Pro Ala Pro Asn
                165                 170

<210>  9
<211>  162
<212>  PRT
<213>  Hepatitis B virus

<400>  9

Gly Gln Asn Leu Ser Thr Ser Asn Pro Leu Gly Phe Phe Pro Asp His
1               5                   10                  15

Gln Leu Asp Pro Ala Phe Arg Ala Asn Thr Ala Asn Pro Asp Trp Asp
            20                  25                  30

Phe Asn Pro Asn Lys Asp Thr Trp Pro Asp Ala Asn Lys Val Gly Ala
        35                  40                  45

Gly Ala Phe Gly Leu Gly Phe Thr Pro Pro His Gly Gly Leu Leu Gly
    50                  55                  60

Trp Ser Pro Gln Ala Gln Gly Ile Leu Gln Thr Leu Pro Thr Asn Pro
65                  70                  75                  80

Pro Pro Ala Ser Thr Asn Arg Gln Ser Gly Arg Gln Pro Thr Pro Leu
                85                  90                  95

Ser Pro Pro Leu Arg Asn Thr His Pro Gln Ala Met Gln Trp Asn Ser
            100                 105                 110

Thr Thr Phe His Gln Thr Leu Gln Asp Pro Arg Val Arg Gly Leu Tyr
        115                 120                 125

Phe Pro Ala Gly Gly Ser Ser Ser Gly Thr Val Asn Pro Val Pro Thr
    130                 135                 140

Thr Val Ser His Ile Ser Ser Ile Phe Ser Arg Ile Gly Asp Pro Ala
145                 150                 155                 160

Leu Asn

<210>  10
<211>  172
<212>  PRT
<213>  Hepatitis B virus

<400>  10

Gly Leu Ser Trp Thr Val Pro Leu Glu Trp Gly Lys Asn His Ser Thr
1               5                   10                  15

Thr Asn Pro Leu Gly Phe Phe Pro Asp His Gln Leu Asp Pro Ala Phe
            20                  25                  30

Arg Ala Asn Thr Arg Asn Pro Asp Trp Asp His Asn Pro Asn Lys Asp
        35                  40                  45

His Trp Thr Ala Ala Asn Lys Val Gly Val Gly Ala Phe Gly Pro Gly
    50                  55                  60

Phe Thr Pro Pro His Gly Gly Leu Leu Gly Trp Ser Pro Gln Ala Gln
65                  70                  75                  80

Gly Met Leu Lys Thr Leu Pro Ala Asp Pro Pro Pro Ala Ser Thr Asn
                85                  90                  95

Arg Gln Ser Gly Arg Gln Pro Thr Pro Ile Thr Pro Pro Leu Arg Asp
            100                 105                 110

Thr His Pro Gln Ala Met Gln Trp Asn Ser Thr Thr Phe His Gln Ala
        115                 120                 125

Leu Gln Asp Pro Arg Val Arg Gly Leu Tyr Phe Pro Ala Gly Gly Ser
    130                 135                 140

Ser Ser Gly Thr Val Asn Pro Val Pro Thr Thr Ala Ser Leu Ile Ser
145                 150                 155                 160

Ser Ile Phe Ser Arg Ile Gly Asp Pro Ala Pro Asn
                165                 170

<210>  11
<211>  173
<212>  PRT
<213>  Hepatitis B virus

<400>  11

Gly Ala Pro Leu Ser Thr Thr Arg Arg Gly Met Gly Gln Asn Leu Ser
1               5                   10                  15

Val Pro Asn Pro Leu Gly Phe Leu Pro Asp His Gln Leu Asp Pro Leu
            20                  25                  30

Phe Arg Ala Asn Ser Ser Ser Pro Asp Trp Asp Phe Asn Lys Ser Lys
        35                  40                  45

Asp Asn Trp Pro Met Ala Asn Lys Val Gly Val Gly Gly Tyr Gly Pro
    50                  55                  60

Gly Phe Thr Pro Pro His Gly Gly Leu Leu Gly Trp Ser Pro Gln Ala
65                  70                  75                  80

Gln Gly Val Leu Thr Thr Leu Pro Ala Asp Pro Pro Pro Ala Ser Thr
                85                  90                  95

Asn Arg Arg Ser Gly Arg Lys Pro Thr Pro Val Ser Pro Pro Leu Arg
            100                 105                 110

Asp Thr His Pro Gln Ala Met Gln Trp Asn Ser Thr Gln Phe His Gln
        115                 120                 125

Ala Leu Leu Asp Pro Arg Val Arg Ala Leu Tyr Phe Pro Ala Gly Gly
    130                 135                 140

Ser Ser Ser Glu Thr Gln Asn Pro Ala Pro Thr Ile Ala Ser Leu Thr
145                 150                 155                 160

Ser Ser Ile Phe Leu Lys Thr Gly Gly Pro Ala Met Asn
                165                 170


<210>  12
<211>  172
<212>  PRT
<213>  Hepatitis B virus

<400>  12

Gly Leu Ser Trp Thr Val Pro Leu Glu Trp Gly Lys Asn Leu Ser Thr
1               5                   10                  15

Ser Asn Pro Leu Gly Phe Leu Pro Asp His Gln Leu Asp Pro Ala Phe

20                  25                  30

Arg Ala Asn Thr Asn Asn Pro Asp Trp Asp Phe Asn Pro Lys Lys Asp
        35                  40                  45

Pro Trp Pro Glu Ala Asn Lys Val Gly Val Gly Ala Tyr Gly Pro Gly
    50                  55                  60

Phe Thr Pro Pro His Gly Gly Leu Leu Gly Trp Ser Pro Gln Ser Gln
65                  70                  75                  80

Gly Thr Leu Thr Thr Leu Pro Ala Asp Pro Pro Pro Ala Ser Thr Asn
                85                  90                  95

Arg Gln Ser Gly Arg Gln Pro Thr Pro Ile Ser Pro Pro Leu Arg Asp
            100                 105                 110

Ser His Pro Gln Ala Met Gln Trp Asn Ser Thr Ala Phe His Gln Ala
        115                 120                 125

Leu Gln Asn Pro Lys Val Arg Gly Leu Tyr Phe Pro Ala Gly Gly Ser
    130                 135                 140

Ser Ser Gly Ile Val Asn Pro Val Pro Thr Ile Ala Ser His Ile Ser
145                 150                 155                 160

Ser Ile Phe Ser Arg Ile Gly Asp Pro Ala Pro Asn
                165                 170

<210>  13
<211>  173
<212>  PRT
<213>  Hepatitis B virus

<400>  13

Gly Ala Pro Leu Ser Thr Ala Arg Arg Gly Met Gly Gln Asn Leu Ser
1               5                   10                  15

Val Pro Asn Pro Leu Gly Phe Phe Pro Asp His Gln Leu Asp Pro Leu
            20                  25                  30

Phe Arg Ala Asn Ser Ser Ser Pro Asp Trp Asp Phe Asn Thr Asn Lys
        35                  40                  45

Asp Asn Trp Pro Met Ala Asn Lys Val Gly Val Gly Gly Phe Gly Pro
    50                  55                  60

Gly Phe Thr Pro Pro His Gly Gly Leu Leu Gly Trp Ser Pro Gln Ala

65                  70                  75                  80

Gln Gly Ile Leu Thr Thr Ser Pro Pro Asp Pro Pro Pro Ala Ser Thr
                85                  90                  95

Asn Arg Arg Ser Gly Arg Lys Pro Thr Pro Val Ser Pro Pro Leu Arg
            100                 105                 110

Asp Thr His Pro Gln Ala Met Gln Trp Asn Ser Thr Gln Phe His Gln
        115                 120                 125

Ala Leu Leu Asp Pro Arg Val Arg Gly Leu Tyr Phe Pro Ala Gly Gly
    130                 135                 140

Ser Ser Ser Glu Thr Gln Asn Pro Ala Pro Thr Ile Ala Ser Leu Thr
145                 150                 155                 160

Ser Ser Ile Phe Ser Lys Thr Gly Asp Pro Ala Met Asn
                165                 170

<210>  14
<211>  30
<212>  PRT
<213>  Artificial Sequence

<220>
<223>  preS fragment

<400>  14

Gly Gly Trp Ser Ser Lys Pro Arg Lys Gly Met Gly Thr Asn Leu Ser
1               5                   10                  15

Val Pro Asn Pro Leu Gly Phe Phe Pro Asp His Gln Leu Asp
            20                  25                  30

<210>  15
<211>  30
<212>  PRT
<213>  Artificial Sequence

<220>
<223>  preS fragment

<400>  15

Leu Gly Phe Phe Pro Asp His Gln Leu Asp Pro Ala Phe Gly Ala Asn
1               5                   10                  15

Ser Asn Asn Pro Asp Trp Asp Phe Asn Pro Ile Lys Asp His
            20                  25                  30

<210>  16
<211>  30
<212>  PRT
<213>  Artificial Sequence

<220>
<223>  preS fragment

<400>  16

Asp Trp Asp Phe Asn Pro Ile Lys Asp His Trp Pro Ala Ala Asn Gln
1               5                   10                  15

Val Gly Val Gly Ala Phe Gly Pro Gly Leu Thr Pro Pro His
            20                  25                  30

<210>  17
<211>  30
<212>  PRT
<213>  Artificial Sequence

<220>
<223>  preS fragment

<400>  17

Ala Phe Gly Pro Gly Leu Thr Pro Pro His Gly Gly Ile Leu Gly Trp
1               5                   10                  15

Ser Pro Gln Ala Gln Gly Ile Leu Thr Thr Val Ser Thr Ile
            20                  25                  30

<210>  18
<211>  30
<212>  PRT
<213>  Artificial Sequence

<220>
<223>  preS fragment

<400>  18

Gln Gly Ile Leu Thr Thr Val Ser Thr Ile Pro Pro Pro Ala Ser Thr
1               5                   10                  15

Asn Arg Gln Ser Gly Arg Gln Pro Thr Pro Ile Ser Pro Pro
            20                  25                  30

<210>  19
<211>  30
<212>  PRT
<213>  Artificial Sequence

<220>
<223>  preS fragment

<400>  19

Gly Arg Gln Pro Thr Pro Ile Ser Pro Pro Leu Arg Asp Ser His Pro
1               5                   10                  15

Gln Ala Met Gln Trp Asn Ser Thr Ala Phe His Gln Ala Leu
            20                  25                  30

<210>  20
<211>  30
<212>  PRT
<213>  Artificial Sequence

<220>
<223>  preS fragment

<400>  20

Trp Asn Ser Thr Ala Phe His Gln Ala Leu Gln Asp Pro Arg Val Arg
1               5                   10                  15

Gly Leu Tyr Phe Pro Ala Gly Gly Ser Ser Ser Gly Thr Val
            20                  25                  30

<210>  21
<211>  33
<212>  PRT
<213>  Artificial Sequence

<220>
<223>  preS fragment

<400>  21

Pro Ala Gly Gly Ser Ser Ser Gly Thr Val Asn Pro Ala Pro Asn Ile
1               5                   10                  15

Ala Ser His Ile Ser Ser Ile Ser Ala Arg Thr Gly Asp Pro Val Thr
            20                  25                  30

Asn

<210>  22
<211>  294
<212>  PRT
<213>  Artificial Sequence

<220>
<223>  PreSF1

<400>  22

Met Val Arg Tyr Thr Thr Glu Gly Gly Thr Lys Thr Glu Ala Glu Asp
1               5                   10                  15

Val Ile Pro Glu Gly Trp Lys Ala Asp Thr Ser Tyr Glu Ser Lys Val
            20                  25                  30

Arg Tyr Thr Thr Glu Gly Gly Thr Lys Thr Glu Ala Glu Asp Val Ile
        35                  40                  45

Pro Glu Gly Trp Lys Ala Asp Thr Ser Tyr Glu Ser Lys Gly Gly Trp
    50                  55                  60

Ser Ser Lys Pro Arg Lys Gly Met Gly Thr Asn Leu Ser Val Pro Asn
65                  70                  75                  80

Pro Leu Gly Phe Phe Pro Asp His Gln Leu Asp Pro Ala Phe Gly Ala
                85                  90                  95

Asn Ser Asn Asn Pro Asp Trp Asp Phe Asn Pro Ile Lys Asp His Trp
            100                 105                 110

Pro Ala Ala Asn Gln Val Gly Val Gly Ala Phe Gly Pro Gly Leu Thr
        115                 120                 125

Pro Pro His Gly Gly Ile Leu Gly Trp Ser Pro Gln Ala Gln Gly Ile
    130                 135                 140

Leu Thr Thr Val Ser Thr Ile Pro Pro Pro Ala Ser Thr Asn Arg Gln
145                 150                 155                 160

Ser Gly Arg Gln Pro Thr Pro Ile Ser Pro Pro Leu Arg Asp Ser His
                165                 170                 175

Pro Gln Ala Met Gln Trp Asn Ser Thr Ala Phe His Gln Ala Leu Gln
            180                 185                 190

Asp Pro Arg Val Arg Gly Leu Tyr Phe Pro Ala Gly Gly Ser Ser Ser
        195                 200                 205

Gly Thr Val Asn Pro Ala Pro Asn Ile Ala Ser His Ile Ser Ser Ile
    210                 215                 220

Ser Ala Arg Thr Gly Asp Pro Val Thr Asn Val Arg Tyr Thr Thr Glu
225                 230                 235                 240

Gly Gly Thr Lys Thr Glu Ala Glu Asp Val Ile Pro Glu Gly Trp Lys
                245                 250                 255

Ala Asp Thr Ser Tyr Glu Ser Lys Val Arg Tyr Thr Thr Glu Gly Gly
            260                 265                 270

Thr Lys Thr Glu Ala Glu Asp Val Ile Pro Glu Gly Trp Lys Ala Asp
        275                 280                 285

Thr Ser Tyr Glu Ser Lys
    290

<210> 23
<211> 298
<212> PRT
<213> Artificial Sequence

<220>
<223> PreSF2

<400> 23

Met Phe Arg Phe Leu Thr Glu Lys Gly Met Lys Asn Val Phe Asp Asp
1               5                   10                  15

Val Val Pro Glu Lys Tyr Thr Ile Gly Ala Thr Tyr Ala Pro Glu Glu
            20                  25                  30

Phe Arg Phe Leu Thr Glu Lys Gly Met Lys Asn Val Phe Asp Asp Val
        35                  40                  45

Val Pro Glu Lys Tyr Thr Ile Gly Ala Thr Tyr Ala Pro Glu Glu Gly
    50                  55                  60

Gly Trp Ser Ser Lys Pro Arg Lys Gly Met Gly Thr Asn Leu Ser Val
65                  70                  75                  80

Pro Asn Pro Leu Gly Phe Phe Pro Asp His Gln Leu Asp Pro Ala Phe
                85                  90                  95

Gly Ala Asn Ser Asn Asn Pro Asp Trp Asp Phe Asn Pro Ile Lys Asp
            100                 105                 110

His Trp Pro Ala Ala Asn Gln Val Gly Val Gly Ala Phe Gly Pro Gly
        115                 120                 125

Leu Thr Pro Pro His Gly Gly Ile Leu Gly Trp Ser Pro Gln Ala Gln
    130                 135                 140

Gly Ile Leu Thr Thr Val Ser Thr Ile Pro Pro Pro Ala Ser Thr Asn
145                 150                 155                 160

Arg Gln Ser Gly Arg Gln Pro Thr Pro Ile Ser Pro Pro Leu Arg Asp
                165                 170                 175

Ser His Pro Gln Ala Met Gln Trp Asn Ser Thr Ala Phe His Gln Ala
            180                 185                 190

Leu Gln Asp Pro Arg Val Arg Gly Leu Tyr Phe Pro Ala Gly Gly Ser
        195                 200                 205

Ser Ser Gly Thr Val Asn Pro Ala Pro Asn Ile Ala Ser His Ile Ser
    210                 215                 220

Ser Ile Ser Ala Arg Thr Gly Asp Pro Val Thr Asn Phe Arg Phe Leu
225                 230                 235                 240

Thr Glu Lys Gly Met Lys Asn Val Phe Asp Asp Val Val Pro Glu Lys
                245                 250                 255

Tyr Thr Ile Gly Ala Thr Tyr Ala Pro Glu Glu Phe Arg Phe Leu Thr
            260                 265                 270

Glu Lys Gly Met Lys Asn Val Phe Asp Asp Val Val Pro Glu Lys Tyr
        275                 280                 285

Thr Ile Gly Ala Thr Tyr Ala Pro Glu Glu
    290                 295

<210>  24
<211>  306
<212>  PRT
<213>  Artificial Sequence

<220>
<223>  PreSF4

<400>  24

Met Gly Lys Ala Thr Thr Glu Glu Gln Lys Leu Ile Glu Asp Val Asn
1               5                   10                  15

Ala Ser Phe Arg Ala Ala Met Ala Thr Thr Ala Asn Val Pro Pro Ala
            20                  25                  30

Asp Lys Gly Lys Ala Thr Thr Glu Glu Gln Lys Leu Ile Glu Asp Val
        35                  40                  45

Asn Ala Ser Phe Arg Ala Ala Met Ala Thr Thr Ala Asn Val Pro Pro
    50                  55                  60

Ala Asp Lys Gly Gly Trp Ser Ser Lys Pro Arg Lys Gly Met Gly Thr
65                  70                  75                  80

Asn Leu Ser Val Pro Asn Pro Leu Gly Phe Phe Pro Asp His Gln Leu

85                  90                  95

Asp Pro Ala Phe Gly Ala Asn Ser Asn Asn Pro Asp Trp Asp Phe Asn
            100                 105                 110

Pro Ile Lys Asp His Trp Pro Ala Ala Asn Gln Val Gly Val Gly Ala
        115                 120                 125

Phe Gly Pro Gly Leu Thr Pro Pro His Gly Gly Ile Leu Gly Trp Ser
    130                 135                 140

Pro Gln Ala Gln Gly Ile Leu Thr Thr Val Ser Thr Ile Pro Pro Pro
145                 150                 155                 160

Ala Ser Thr Asn Arg Gln Ser Gly Arg Gln Pro Thr Pro Ile Ser Pro
                165                 170                 175

Pro Leu Arg Asp Ser His Pro Gln Ala Met Gln Trp Asn Ser Thr Ala
            180                 185                 190

Phe His Gln Ala Leu Gln Asp Pro Arg Val Arg Gly Leu Tyr Phe Pro
        195                 200                 205

Ala Gly Gly Ser Ser Ser Gly Thr Val Asn Pro Ala Pro Asn Ile Ala
    210                 215                 220

Ser His Ile Ser Ser Ile Ser Ala Arg Thr Gly Asp Pro Val Thr Asn
225                 230                 235                 240

Gly Lys Ala Thr Thr Glu Glu Gln Lys Leu Ile Glu Asp Val Asn Ala
                245                 250                 255

Ser Phe Arg Ala Ala Met Ala Thr Thr Ala Asn Val Pro Pro Ala Asp
            260                 265                 270

Lys Gly Lys Ala Thr Thr Glu Glu Gln Lys Leu Ile Glu Asp Val Asn
        275                 280                 285

Ala Ser Phe Arg Ala Ala Met Ala Thr Thr Ala Asn Val Pro Pro Ala
    290                 295                 300

Asp Lys
305

## Description

The present invention relates to a fusion protein for use in the treatment and/or prevention of a hepatitis B virus (HBV) infection.

Hepatitis B is a liver disease caused by hepatitis B viruses. The disease affects millions of people per year throughout the world. The HBV is present in the blood and body fluids of infected people and can therefore be spread by contacting these fluids with fluids of healthy people.

HBV primarily interferes with the functions of the liver by replicating in liver cells. During HBV infection, the host immune response causes both hepatocellular damage and viral clearance.

Acute HBV infections are usually not treated because most people are able to clear the infection spontaneously. However, chronic HBV infections have to be treated in order to reduce the risk of cirrhosis and liver cancer. Antiviral drugs currently used in the treatment of HBV infections include lamivudine, adefovir, tenofovir, telbivudine and entecavir. Furthermore, interferon alpha-2a acting as immune system modulator can also be used in the treatment. However, none of these drugs can clear HBV infections. These drugs can only stop the HBV from replicating, thus minimizing liver damage.

It is an object of the present invention to provide new means in the treatment and/or prevention of HBV infections which overcome the drawbacks of the present HBV treatments. A particularly important objective is viral clearance of HBV in chronically infected patients by restoring an efficient humoral and cellular immune response.

These objectives are achieved by a fusion protein for use in the treatment and/or prevention of a hepatitis B virus infection comprising at least one hepatitis B PreS polypeptide or fragment thereof fused to at least one peptide consisting of an amino acid sequence having at least 80% identity to a sequence selected from the group consisting of SEQ ID No. 1, SEQ ID No. 2, SEQ ID No. 3 and SEQ ID No. 4.

It surprisingly turned out that a fusion protein comprising a hepatitis B PreS polypeptide or fragment thereof and at least one peptide consisting of an amino acid sequence having at least 80% identity to a sequence selected from the group consisting of SEQ ID No. 1, SEQ ID No. 2, SEQ ID No. 3 and SEQ ID No. 4 induces the formation of PreS specific antibodies in an individual to a much higher extent compared to PreS alone or other fusion proteins comprising peptides different from SEQ ID No. 1, SEQ ID No. 2, SEQ ID No. 3 and SEQ ID No. 4. Furthermore the antibodies produced in response to the administration of the fusion protein of the present invention show superior hepatitis B neutralizing effects and are able to inhibit hepatitis B virus infections.

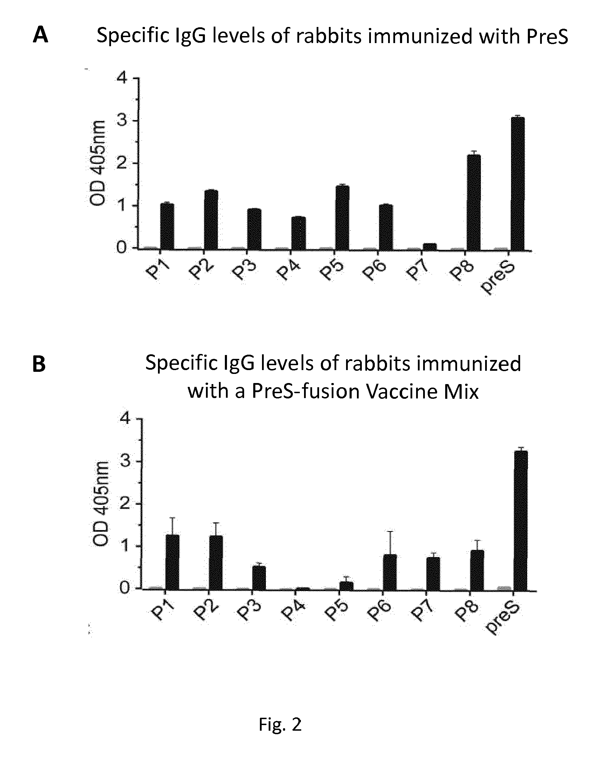

As shown in Fig. 2B the administration of the fusion protein of the present invention results in the formation of antibodies which are specifically directed to the first 30 (peptide P1) and 50 (peptide P2) amino acid residues of HBV PreS, to a lower extent to the C-terminal region (peptides P6 to P8) and to a negligible extent to the central part of HBV PreS (peptides P4 and P5). Since the N-terminal part of HBV PreS is known to play an important role in liver cell attachment of HBV and HBV infections antibodies directed to this part of the PreS polypeptide are particularly useful in the treatment and/or prevention of HBV infections. In contrast thereto, the sole administration of HBV PreS does not show these effects. The antibodies produced thereby are able to bind to almost any part of HBV PreS (see Fig. 2A). This shows that the immune response induced by the fusion proteins of the present invention is more focused on those parts of the HBV PreS polypeptide which are involved in the HBV infection.

The fusion protein of the present invention may comprise one or more hepatitis B PreS polypeptides or one or more fragments thereof. The presence of more than one hepatitis B PreS polypeptides or fragments thereof in the fusion protein has the advantage that more antigens are presented to the immune system allowing the formation of even more antibodies directed to PreS. In a particularly preferred embodiment of the present invention the fusion protein comprises one, two, three, four, five, six, seven, eight, nine or ten hepatitis B PreS polypeptides or fragments thereof. The HBV PreS polypeptides as well as their fragments as defined herein being part of the fusion protein of the present invention may be derived from the same HBV genotype or from different genotypes. For instance, the fusion protein of the present invention may comprise the PreS polypeptide or a fragment thereof of HBV genotype A only or may be combined with a further PreS polypeptide or fragment thereof derived from HBV genotype B, C, D, E, F, G or H.

In a particularly preferred embodiment of the present invention the fusion protein comprises at least one peptide consisting of an amino acid sequence having at least 80% identity to SEQ ID No. 1, at least one peptide consisting of an amino acid sequence having at least 80% identity to SEQ ID No. 2, at least one peptide consisting of an amino acid sequence having at least 80% identity to SEQ ID No. 3 and at least one peptide consisting of an amino acid sequence having at least 80% identity to SEQ ID No. 4. Alternatively the fusion protein of the present invention may comprise one, two, three, four, five six, seven, eight, nine or ten of these peptides in any possible combination or even only one specific peptide in the same amount.

The terms "fused to" or "fusion protein", as used herein, refer to a protein comprising a hepatitis B PreS polypeptide or fragment thereof that are expressed and prepared as one single recombinant polypeptide chain.

Methods for the production of fusion proteins are well known in the art and can be found in standard molecular biology references such as Sambrook et al. (Molecular Cloning, 2nd ed., Cold Spring Harbor Laboratory Press, 1989) and Ausubel et al. (Short Protocols in Molecular Biology, 3rd ed; Wiley and Sons, 1995). In general, a fusion protein is produced by first constructing a fusion gene which is inserted into a suitable expression vector, which is, in turn, used to transfect a suitable host cell. In general, recombinant fusion constructs are produced by a series of restriction enzyme digestions and ligation reactions which result in the desired sequences being incorporated into a plasmid. If suitable restriction sites are not available, synthetic oligonucleotide adapters or linkers can be used as is known by those skilled in the art and described in the references cited above. The polynucleotide sequences encoding allergens and native proteins can be assembled prior to insertion into a suitable vector or the sequence encoding the allergen can be inserted adjacent to a sequence encoding a native sequence already present in a vector. Insertion of the sequence within the vector should be in frame so that the sequence can be transcribed into a protein. It will be apparent to those of ordinary skill in the art that the precise restriction enzymes, linkers and/or adaptors required as well as the precise reaction conditions will vary with the sequences and cloning vectors used. The assembly of DNA constructs, however, is routine in the art and can be readily accomplished by a person skilled in the art.

A fragment of a hepatitis B PreS polypeptide consists preferably of at least 30, preferably at least 40, more preferably at least 50, consecutive amino acid residues and may comprise PreS1 and/or PreS2 of the hepatitis B PreS polypeptide. In a particularly preferred embodiment of the present invention a fragment of a hepatitis B PreS polypeptide may comprise amino acid residues 1 to 70, preferably amino acid residues 1 to 65, more preferably amino acid residues 1 to 60, more preferably amino acid residues 1 to 55, more preferably amino acid residues 1 to 50, more preferably 1 to 45, more preferably amino acid residues 1 to 40, more preferably amino acid residues 1 to 35, more preferably amino acid residues 5 to 70, more preferably amino acid residues 5 to 65, more preferably amino acid residues 5 to 60, more preferably amino acid residues 5 to 55, more preferably amino acid residues 5 to 50, more preferably 5 to 45, more preferably amino acid residues 5 to 40, more preferably amino acid residues 5 to 35, more preferably amino acid residues 10 to 70, more preferably amino acid residues 10 to 65, more preferably amino acid residues 10 to 60, more preferably amino acid residues 10 to 55, more preferably amino acid residues 10 to 50, more preferably 10 to 45, more preferably amino acid residues 10 to 40, more preferably amino acid residues 10 to 35, more preferably amino acid residues 15 to 70, more preferably amino acid residues 15 to 65, more preferably amino acid residues 15 to 60, more preferably amino acid residues 15 to 55, more preferably amino acid residues 15 to 50, more preferably 15 to 45, more preferably amino acid residues 15 to 40, more preferably amino acid residues 15 to 35, of the hepatitis B PreS polypeptide, preferably of the HBV PreS polypeptides consisting of SEQ ID Nos. 5, 7, 8, 9, 10, 11, 12, 13 or 14, whereby SEQ ID Nos. 8 to 14 belong to HBV genotypes B to H, respectively.

The at least one peptide to be fused to at least one hepatitis B PreS polypeptide or fragment thereof has an identity of at least 80%, preferably of at least 85%, more preferably of at least 90%, more preferably of at least 92%, more preferably of at least 94%, more preferably of at least 96%, more preferably of at least 98%, more preferably of at least 99%, in particular of 100%, to SEQ ID No. 1, SEQ ID No. 2, SEQ ID No. 3 and SEQ ID No. 4. The degree of identity of a first amino acid sequence to a second amino acid can be determined by a direct comparison between both amino acid sequences using certain algorithms. Sequence identity is preferably determined by BLAST alignment (http://blast.ncbi.nlm.nih. gov/; Altschul SF et al J. Mol. Bi o1. 215 (1990): 403-410) using the BLOSUM62 matrix, a gap existence penalty of 11, and a gap extension penalty of 1.

According to a preferred embodiment of the present invention the amino acid sequence of the PreS polypeptide is at least 80% identical to SEQ ID No. 5, SEQ ID No. 7, SEQ ID No. 8, SEQ ID No. 9, SEQ ID No. 10, SEQ ID No. 11, SEQ ID No. 12 or SEQ ID No. 13, most preferably to SEQ ID No. 5.

The hepatitis B PreS polypeptide to be fused to at least one of the peptides described above has an identity of at least 80%, preferably of at least 85%, more preferably of at least 90%, more preferably of at least 92%, more preferably of at least 94%, more preferably of at least 96%, more preferably of at least 98%, more preferably of at least 99%, in particular of 100%, to SEQ ID No. 5, SEQ ID No. 7, SEQ ID No. 8, SEQ ID No. 9, SEQ ID No. 10, SEQ ID No. 11, SEQ ID No. 12 or SEQ ID No. 13.

According to a further preferred embodiment of the present invention the at least one peptide is fused to the N- and/or C-terminus of the PreS polypeptide.

"Fused to the N- and/or C-terminus", as used herein, means that at least one peptide is fused to the N- and/or C-terminus of the PreS polypeptide or fragment thereof. The fusion protein of the present invention may comprise one or more peptides fused to the N-terminus of the PreS polypeptide or fragment thereof or to its C-terminus.

According to a preferred embodiment of the present invention the fusion protein comprises an amino acid sequence which is at least 80% identical to SEQ ID No. 6.

The fusion protein of the present invention has an identity of at least 80%, preferably of at least 85%, more preferably of at least 90%, more preferably of at least 92%, more preferably of at least 94%, more preferably of at least 96%, more preferably of at least 98%, more preferably of at least 99%, in particular of 100%, to SEQ ID No. 6.

According to a further preferred embodiment of the present invention the hepatitis B virus infection is caused by a hepatitis B virus genotype A, B, C, D, E, F, G, H or a subtype therof. It is preferred to use a HBV PreS polypeptide or fragment thereof of the same genotype to treat and/or prevent a HBV caused by this HBV genotype (e.g. PreS of HBV genotype A is used to treat/prevent an infection of HBV genotype A or one of its subtypes). Due to the conserved amino acid sequences in those parts of the PreS polypeptide which is known to be involved in the HBV infection, it is of course also possible to use a HBV PreS polypeptide or fragment thereof of one genotype to treat/prevent an infection of another HBV genotype (e.g. PreS of HBV genotype A is used to treat/prevent an infection of HBV genotype B, C, D, E, F, G and/or H or a subtype thereof).

The fusion protein of the present invention may be used in the treatment and/or prevention of HBV infections of various genotypes and subtypes thereof. Subtypes of hepatitis B viruses include A1, A2, A3, A4, A5, B1, B2, B3, B4, B5, C1, C2, C3, C4, C5, D1, D2, D3, D4, D5, F1, F2, F3 and F4 as discussed in Schaefer et al. (World J Gastroenterol 13(2007):14-21).

According to a particularly preferred embodiment of the present invention the fusion protein is administered to an individual at least once in an amount of 0.01 µg/kg body weight to 5 mg/kg body weight, preferably 0.1 µg/kg body weight to 2 mg/kg body weight. According to further preferred embodiment of the present invention the fusion protein is administered to a patient in an amount of 5 to 50 µg, preferably 10 to 40 µg, more preferably 15 to 30 µg, either independent from the body weight (i.e. a dose may comprise 15, 20, 25 or 30 µg) or per kg body weight.

The amount of fusion protein that may be combined with excipients to produce a single dosage form will vary depending upon the host treated and the particular mode of administration. The dose of the fusion protein may vary according to factors such as the disease state, age, sex and weight of the individual, and the ability to elicit the desired antibody response in the individual. Dosage regime may be adjusted to provide the optimum therapeutic response. For example, several divided doses may be administered daily or the dose may be proportionally reduced as indicated by the exigencies of the therapeutic situation. The dose of the vaccine may also be varied to provide optimum preventative dose response depending upon the circumstances. For instance, the polypeptides and vaccine of the present invention may be administered to an individual at intervals of several days, one or two weeks or even months depending always on the level of hepatitis B PreS specific IgG induction.

In a preferred embodiment of the present invention the fusion protein of the present invention is applied between 2 and 10, preferably between 2 and 7, even more preferably up to 5 and most preferably up to 3 times. In a particularly preferred embodiment the time interval between the subsequent vaccinations is chosen to be between 2 weeks and 5 years, preferably between 1 month and up to 3 years, more preferably between 2 months and 1.5 years. The repeated administration of the fusion protein of the present invention may maximize the final effect of the treatment.

According to a further preferred embodiment of the present invention the fusion protein is administered together with at least one adjuvant and/or pharmaceutical acceptable excipient.

The fusion protein of the present invention can be administrated subcutaneously, intramuscularly, intravenously, mucosally etc. Depending on the dosage form and administration route the fusion protein of the present invention may be combined with excipients, diluents, adjuvants and/or carriers. A preferred adjuvant is alum. Suitable protocols for the production of vaccine formulations are known to the person skilled in the art and can be found e.g. in "Vaccine Protocols" (A. Robinson, M. P. Cranage, M. Hudson; Humana Press Inc., U. S.; 2nd edition 2003).

The fusion protein of the present invention may be formulated also with other adjuvants regularly used in vaccines. For instance, suitable adjuvants may be MF59, aluminum phosphate, calcium phosphate, cytokines (e.g. IL-2, IL-12, GM-CSF), saponins (e.g. QS21), MDP derivatives, CpG oligonucleotides, LPS, MPL, polyphosphazenes, emulsions (e.g. Freund's, SAF), liposomes, virosomes, iscoms, cochleates, PLG microparticles, poloxamer particles, virus-like particles, heat-labile enterotoxin (LT), cholera toxin (CT), mutant toxins (e.g. LTK63 and LTR72), microparticles and/or polymerized liposomes. Suitable adjuvants are commercially available as, for example, AS01B (MPL and QS21 in a liposome formulation), AS02A, AS15, AS-2, AS-03 and derivatives thereof (GlaxoSmithKline, USA); CWS (cell-wall skeleton), TDM (trehalose-6,6'-dimycolate), LeIF (Leishmania elongation initiation factor), aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7 or -12 may also be used as adjuvants. Preferred adjuvants for use in eliciting a predominantly Thl-type response include, for example, a combination of monophosphoryl lipid A, preferably 3-O-deacylated monophosphoryl lipid A (3D-MPL), optionally with an aluminum salt. Aqueous formulations comprising monophosphoryl lipid A and a surfactant have been described in WO 98/43670.

Another preferred adjuvant is a saponin or saponin mimetics or derivatives, preferably QS21 (Aquila Biopharmaceuticals Inc.), which may be used alone or in combination with other adjuvants. For example, an enhanced system involves the combination of a monophosphoryl lipid A and saponin derivative, such as the combination of QS21 and 3D-MPL. Other preferred formulations comprise an oil-in-water emulsion and tocopherol. A particularly potent adjuvant formulation is QS21, 3D-MPL and tocopherol in an oil-in-water emulsion. Additional saponin adjuvants for use in the present invention include QS7 (described in WO 96/33739 and WO 96/11711) and QS17 (described in US 5,057,540 and EP 0 362 279 B1).

The SEQ ID Nos. disclosed herein have the following amino acid sequence (Genbank Acc. No.:

| **SEQ ID No.** | **Amino acid sequence** | **Genbank Acc. No.** |
|---|---|---|
| 1 | EAAFNDAIKASTGGAYESYKFIPALEAAVK | |
| 2 | AEEVKVIPAGELQVIEKVDAAFKVAATAANAAPA NDK | |
| 3 | ADLGYGPATPAAPAAGYTPATPAAPAEAAPAGK | |
| 4 | ATTEEQKLIEKINAGFKAALAAAAGVQPADKYR | |
| 5 | GGWSSKPRKGMGTNLSVPNPLGFFPDHQLDPAFG ANSNNPDWDFNPIKDHWPAANQVGVGAFGPGLTP PHGGILGWSPQAQGILTTVSTIPPPASTNRQSGR QPTPISPPLRDSHPQAMQWNSTAFHQALQDPRVR GLYFPAGGSSSGTVNPAPNIASHISSISARTGDP VTN | AAT28735.1 |
| 6 | MEAAFNDAIKASTGGAYESYKFIPALEAAVKAEE VKVIPAGELQVIEKVDAAFKVAATAANAAPANDK GGWSSKPRKGMGTNLSVPNPLGFFPDHQLDPAFG ANSNNPDWDFNPIKDHWPAANQVGVGAFGPGLTP PHGGILGWSPQAQGILTTVSTIPPPASTNRQSGR QPTPISPPLRDSHPQAMQWNSTAFHQALQDPRVR GLYFPAGGSSSGTVNPAPNIASHISSISARTGDP VTNADLGYGPATPAAPAAGYTPATPAAPAEAAPA GKATTEEQKLIEKINAGFKAALAAAAGVQPADKY R | |
| 7 | GGWSSKPRKGMGTNLSVPNPLGFFPDHQLDPAFK ANSDNPDWDLNPHKDNWPDSNKVGVGAFGLGFTP PHGGLLGWSPQAQGILTTVPAAPPPASTNRQVAR PPTPLSPPLRDTHPQAMQWNSTTFHQTLQDPRVR ALYFPAGGSSSGTVNPVQNTASSISSILSKTGDP VPN | BAE80758.1 |
| 8 | GGYSSKPRKGMGTNLSVPNPLGFLPDHQLDPAFG ANSNNPDWDFNPNKDPWPEAWQVGVGAFGPGFTP PHGGLLGWSPQAQGILTPVPAAPPPASTNRQSGR QPTPISPPLRDSHPQAMQWNSTTFHQALLDPRVR GLYFPAGGSSSGTTNPVPTTASPISSIFSRTGDP APN | ADA56873.1 |
| 9 | GQNLSTSNPLGFFPDHQLDPAFRANTANPDWDFN PNKDTWPDANKVGAGAFGLGFTPPHGGLLGWSPQ AQGILQTLPTNPPPASTNRQSGRQPTPLSPPLRN THPQAMQWNSTTFHQTLQDPRVRGLYFPAGGSSS GTVNPVPTTVSHISSIFSRIGDPALN | AF121240.1 |
| 10 | GLSWTVPLEWGKNHSTTNPLGFFPDHQLDPAFRA NTRNPDWDHNPNKDHWTAANKVGVGAFGPGFTPP HGGLLGWSPQAQGMLKTLPADPPPASTNRQSGRQ PTPITPPLRDTHPQAMQWNSTTFHQALQDPRVRG LYFPAGGSSSGTVNPVPTTASLISSIFSRIGDPA PN | AAW79851.1 |
| 11 | GAPLSTTRRGMGQNLSVPNPLGFLPDHQLDPLFR | AAM09056.1 |
| | ANSSSPDWDFNKSKDNWPMANKVGVGGYGPGFTP PHGGLLGWSPQAQGVLTTLPADPPPASTNRRSGR KPTPVSPPLRDTHPQAMQWNSTQFHQALLDPRVR ALYFPAGGSSSETQNPAPTIASLTSSIFLKTGGP AMN | |
| 12 | GLSWTVPLEWGKNLSTSNPLGFLPDHQLDPAFRA NTNNPDWDFNPKKDPWPEANKVGVGAYGPGFTPP HGGLLGWSPQSQGTLTTLPADPPPASTNRQSGRQ PTPISPPLRDSHPQAMQWNSTAFHQALQNPKVRG LYFPAGGSSSGIVNPVPTIASHISSIFSRIGDPA PN | AB064313.1 |
| 13 | GAPLSTARRGMGQNLSVPNPLGFFPDHQLDPLFR ANSSSPDWDFNTNKDNWPMANKVGVGGFGPGFTP PHGGLLGWSPQAQGILTTSPPDPPPASTNRRSGR KPTPVSPPLRDTHPQAMQWNSTQFHQALLDPRVR GLYFPAGGSSSETQNPAPTIASLTSSIFSKTGDP AMN | FJ356716.1 |

The present invention is further illustrated by the following figures and examples, however, without being restricted thereto.

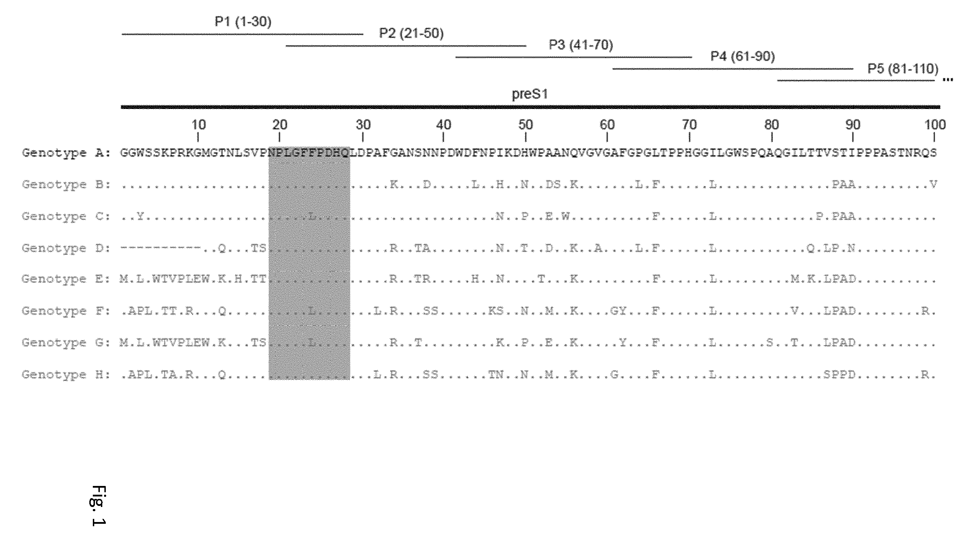

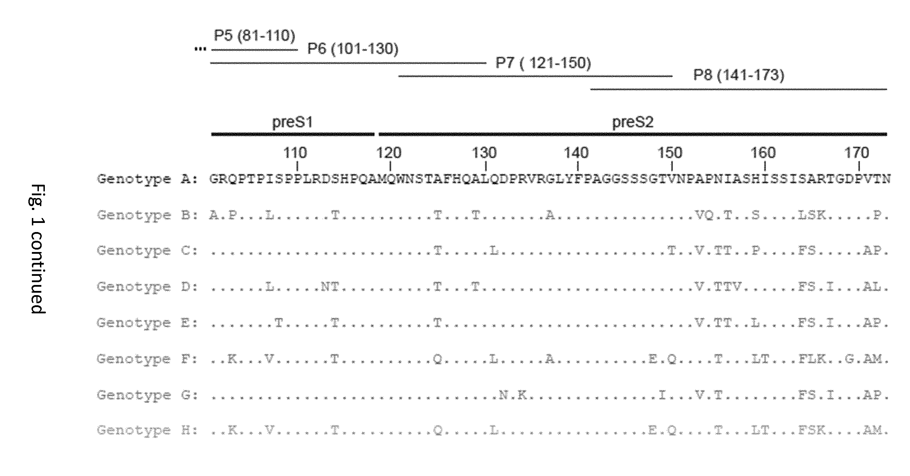

Fig. 1 shows the allocation of PreS peptides to aligned PreS sequences from different genotypes. Identical amino acids are indicated by points, the PreS1 domain includes amino acid residues 1 to 118 and the PreS2 domain amino acid residues 119 to 173 (see also SEQ ID No. 5) and amino acid residues 19 to 28 (grey box) play a crucial role in liver cell attachment of HBV and infection.
Fig. 2 shows IgG responses of rabbits immunized with PreS (n=1; Fig. 2A), or 20µg of a PreS-fusion vaccine Mix (n=2; Fig. 2B), before (left bars in grey) and after (right bars in black) immunization.. Optical density values (y-axes: OD values at 405nm) correspond to IgG levels towards PreS and PreS-derived synthetic overlapping peptides P1-P8 (x-axes). Results represent mean values with SD from triplicate determinations.

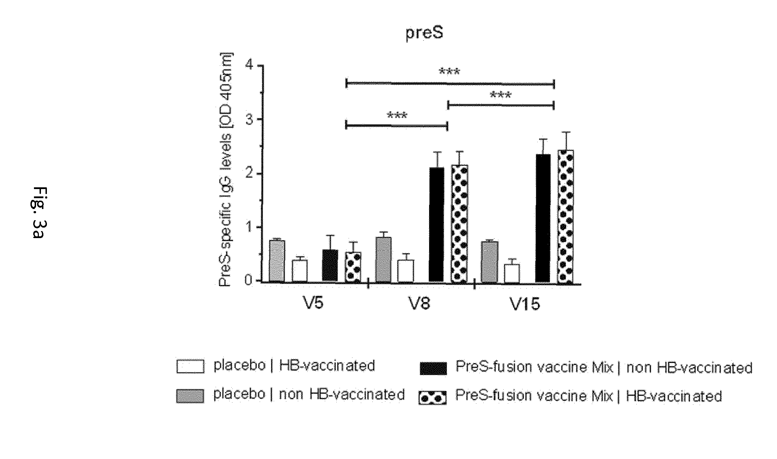

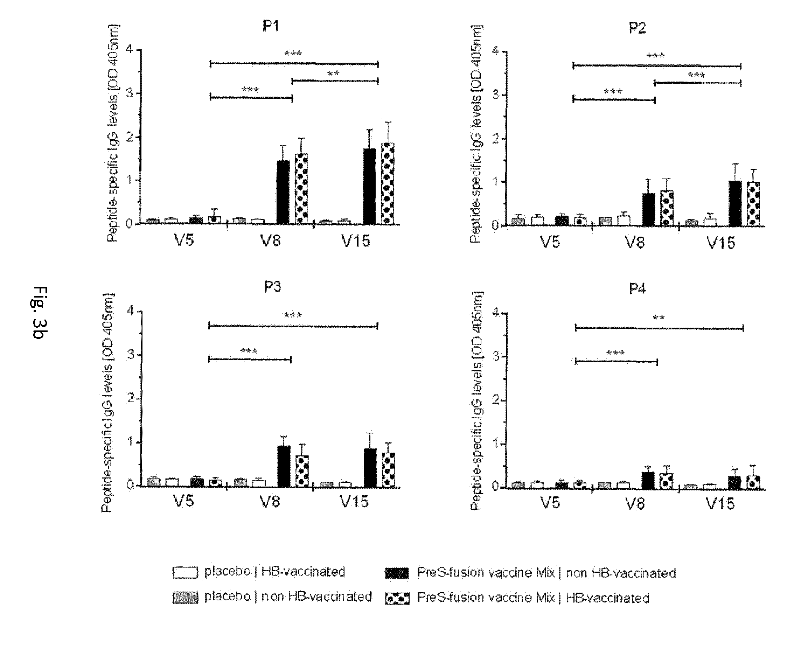

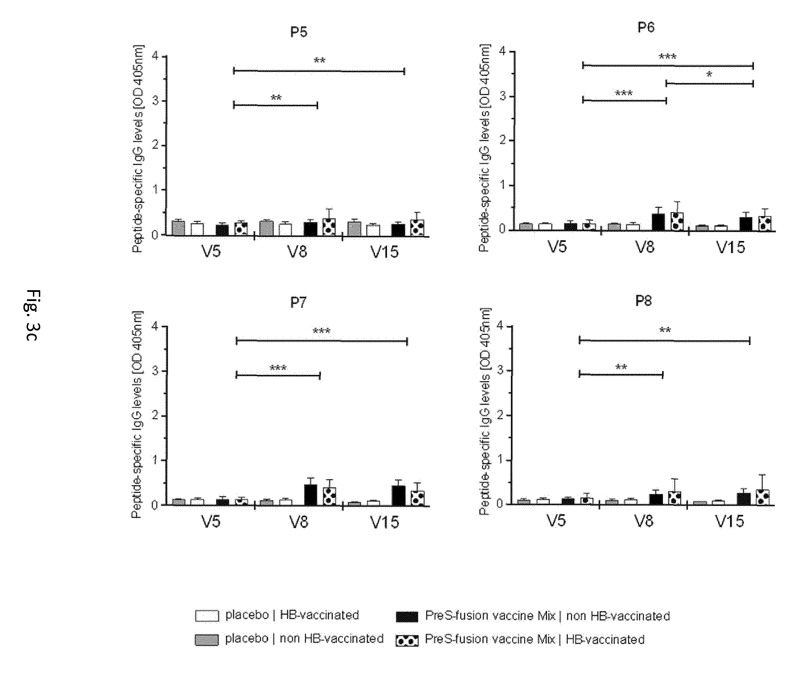

Figs. 3A to 3C show IgG responses towards PreS (Fig. 3A) and synthetic PreS-derived overlapping peptides P1-P8 (Figs. 3B, and 3C) of subjects vaccinated with PreS-fusion vaccine Mix or placebo. Shown are optical density values (y-axes: OD values, means of triplicate determinations) corresponding to IgG levels towards PreS and peptides P1-P8 measured in subjects with or without prior hepatitis B vaccination who had been immunized with PreS-fusion vaccine Mix (n=22) or placebo (n=8) before (V5) and at different time points after immunization (V8 and V15) (x-axes). Results are represented as mean values with SD and significant differences (in all PreS-fusion vaccine Mix-vaccinated individuals at V5, V8, and V15) are indicated: *p < 0.05, **p < 0.01, ***p < 0.001.

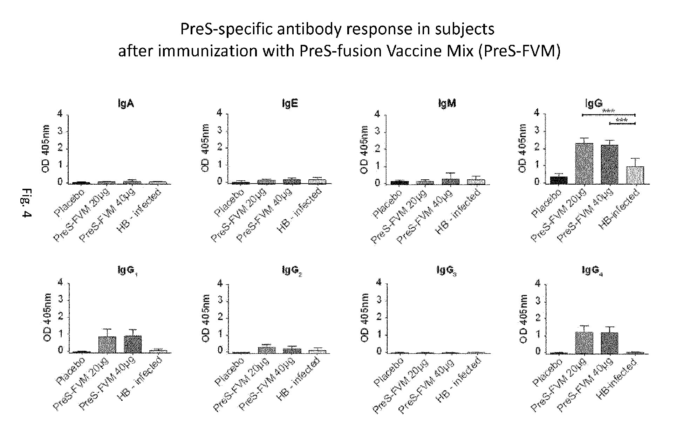

Fig. 4 shows PreS-specific antibody responses of subjects vaccinated with PreS-fusion vaccine Mix (PreS-FVM) or placebo and antibodies present in hepatitis B-infected individuals. Shown are optical density values (y-axes: OD values) corresponding to IgA, IgE, IgM, IgG and IgG subclass (IgG1-IgG4) levels specific for PreS of subjects immunized with placebo (n=8), 20µg (n=10) or 40µg of PreS-fusion vaccine Mix (n=12) as well as of hepatitis B-infected individuals (n=19) (x-axes). Graphs show mean values with SD. Significant differences are indicated: ***p < 0.001.

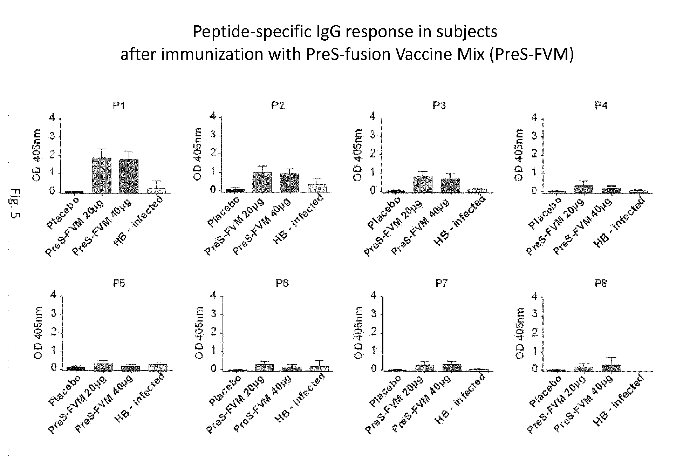

Fig. 5 shows IgG responses specific for PreS peptides P1-P8 of subjects vaccinated with PreS-fusion vaccine Mix (PreS-FVM) or placebo and IgG present in hepatitis B-infected individuals. Shown are optical density values (y-axes: OD values) corresponding to IgG levels specific for PreS-derived peptides (P1-P8) of subjects immunized with placebo (n=8), 20µg (n=10) or 40µg of PreS vaccine mixture (n=12) at V15 as well as of hepatitis B-infected individuals (n=19) (x-axes). Results are represented as mean values with SD.

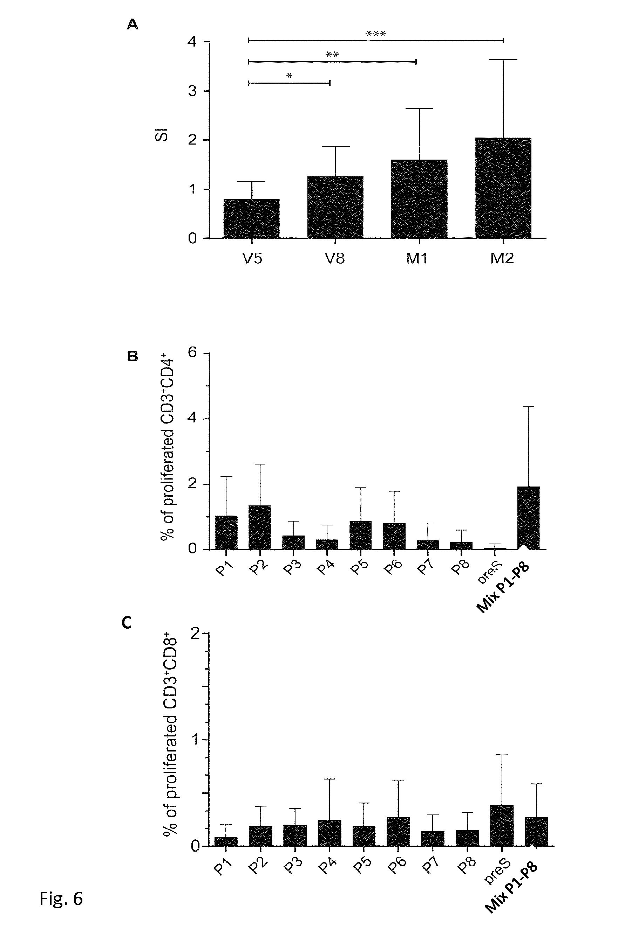

Fig. 6 shows PreS- and peptide-specific T cell responses. Fig.6A: PreS-specific PBMC proliferations (y-axis: stimulation indices SIs) assessed by [3H] thymidine incorporation in subjects immunized with PreS-fusion vaccine Mix (n=19) at different time points (x-axis). Mean values with SD and significant differences are indicated: *p < 0.05, **p < 0.01, ***p<0.001. Fig. 6B, and Fig 6C: Percentages of proliferated CD4 (B) and CD8 (C) T cells (y-axes) after stimulation with PreS peptides (P1-P8), PreS or an equimolar peptide mix (x-axes) in blood samples of subjects immunized with PreS-fusion vaccine Mix (n=11) at time point M2. Results are represented as mean values with SD.

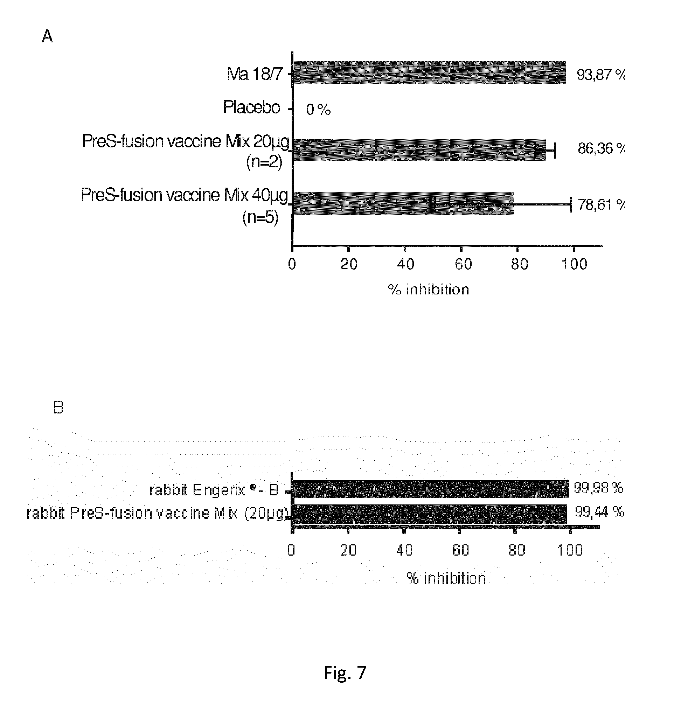

Fig. 7 shows the antibody-induced inhibition of hepatitis B virus infection in an in-vitro virus neutralization assay which is based on in-vitro cultured liver cells. Percentages of the inhibition of hepatitis B infection of cultured HepG2-hNTCP (x-axis) achieved by pre-incubation of virus with anti-sera containing virus neutralizing antibodies. Fig. 7A: Inhibition of virus infection by Ma 18/7 (Positive control), serum from a placebo-treated human subject, sera from human subjects after immunization with PreS-fusion vaccine Mix (n=7), all subjects without prior hepatitis B vaccination. Fig. 7B: Inhibition of virus infection by sera from rabbits immunized with the commercial hepatitis B vaccine Engerix or the PreS-fusion vaccine Mix.

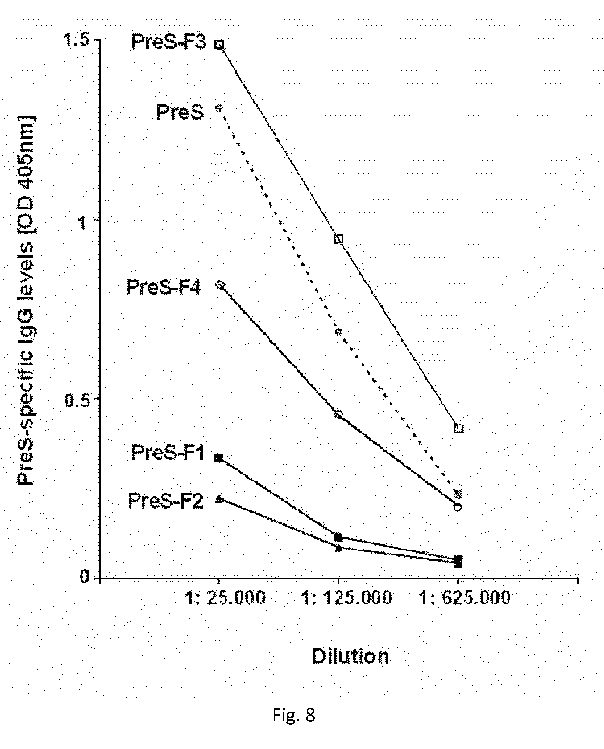

Fig. 8 shows a comparison of total serum IgG towards PreS in sera of New Zealand White (NZW) rabbits which have undergone immunization, either with recombinant PreS or PreS-fusion-proteins (PreS-F1 - PreS-F4), as emulsion in Complete Freund's Adjuvant. The x-axis indicates the dilution of sera and on the y-axis, the OD values, measured at 405 nm are depicted. The experiment was assayed in duplicates.

### EXAMPLES:

### Example 1: Expression and purification of recombinant PreS, synthesis of PreS overlapping peptides, sequence alignments

Expression and purification of a hexahistidine-tagged recombinant PreS protein (PreS1+PreS2 (SEQ ID No. 5; genotype A; subtype adw2, derived from GenBank: AAT28735.1) in Escherichia coli BL21 (DE3, Stratagene, USA) has been performed as described in Niespodziana K et al. (J Allergy Clin Immunol 127(2011):1562-70).

Eight peptides of a length of approximately 30 amino acids and an overlap of 10 amino acids spanning the complete sequence of PreS (genotype A, subtype adw2; Table A; Fig. 1) were synthesized by a Fmoc (9-fluorenylmethoxycarbonyl)-strategy with HBTU [2-(1H-Benzotriazol-1-yl)1,1,3,3 tetramethyluronium hexafluorophosphat] activation (CEM-Liberty, Matthews, NC; Applied Biosystems, Life technologies, USA).

**Table A:**

| Peptide | Sequence | SEQ ID No. |
|---|---|---|
| P1 | GGWSSKPRKGMGTNLSVPNPLGFFPDHQLD | 14 |
| P2 | LGFFPDHQLDPAFGANSNNPDWDFNPIKDH | 15 |
| P3 | DWDFNPIKDHWPAANQVGVGAFGPGLTPPH | 16 |
| P4 | AFGPGLTPPHGGILGWSPQAQGILTTVSTI | 17 |
| P5 | QGILTTVSTIPPPASTNRQSGRQPTPISPP | 18 |
| P6 | GRQPTPISPPLRDSHPQAMQWNSTAFHQAL | 19 |
| P7 | WNSTAFHQALQDPRVRGLYFPAGGSSSGTV | 20 |
| P8 | PAGGSSSGTVNPAPNIASHISSISARTGDPVTN | 21 |

(overlapping regions of the peptides are underlined)

Peptides were purified by preparative HPLC and their identity was confirmed by mass spectrometry (Microflex MALDI-TOF, Bruker, USA).

An alignment of the PreS genotype A, serotype adw2 sequence and peptide sequences thereof with HBV genotypes B-H was performed with CLUSTAL W using reference sequences from the HBV data base (HBVdb: https://hbvdb.ibcp.fr/HBVdb/HBVdbIndex) (Hayer J et al. Nucleic Acids Res 2012;gks1022) (see Fig. 1).

### Example 2: Immunization of rabbits

Specific rabbit antibodies against recombinant PreS were raised by immunization of a New Zealand white rabbit with purified PreS (200 µg per injection) using Freund's complete adjuvant (CFA) for the first and incomplete Freund's adjuvant (IFA) for the second and third injection (Charles River, Germany). In addition, New Zealand white rabbits were immunized three times with a mix containing 20µg (n=2) or 40µg (n=2) of each of the four PreS vaccine mixture components (PreS vaccine mixture-20 / PreS vaccine mixture-40) using Al(OH)₃ as adjuvant. The four PreS vaccine mixture components include PreS fusion proteins PreSF1, PreSF2, PreSF3 and PreSF4 having the following amino acid sequences:

PreSF1 (SEQ ID No. 22):

MVRYTTEGGTKTEAEDVIPEGWKADTSYESKVRYTTEGGTKTEAEDVIPEGWKADT
SYESKGGWSSKPRKGMGTNLSVPNPLGFFPDHQLDPAFGANSNNPDWDFNPIKDHW
PAANQVGVGAFGPGLTPPHGGILGWSPQAQGILTTVSTIPPPASTNRQSGRQPTPI
SPPLRDSHPQAMQWNSTAFHQALQDPRVRGLYFPAGGSSSGTVNPAPNIASHISSI

SARTGDPVTNVRYTTEGGTKTEAEDVIPEGWKADTSYESKVRYTTEGGTKTEAEDV
IPEGWKADTSYESK

PreSF2 (SEQ ID No. 23):

MFRFLTEKGMKNVFDDVVPEKYTIGATYAPEEFRFLTEKGMKNVFDDVVPEKYTIG
ATYAPEEGGWSSKPRKGMGTNLSVPNPLGFFPDHQLDPAFGANSNNPDWDFNPIKD
HWPAANQVGVGAFGPGLTPPHGGILGWSPQAQGILTTVSTIPPPASTNRQSGRQPT
PISPPLRDSHPQAMQWNSTAFHQALQDPRVRGLYFPAGGSSSGTVNPAPNIASHIS
SISARTGDPVTNFRFLTEKGMKNVFDDVVPEKYTIGATYAPEEFRFLTEKGMKNVF
DDVVPEKYTIGATYAPEE

PreSF3 (SEQ ID No. 6):

MEAAFNDAIKASTGGAYESYKFIPALEAAVKAEEVKVIPAGELQVIEKVDAAFKVA
ATAANAAPANDKGGWSSKPRKGMGTNLSVPNPLGFFPDHQLDPAFGANSNNPDWDF
NPIKDHWPAANQVGVGAFGPGLTPPHGGILGWSPQAQGILTTVSTIPPPASTNRQS
GRQPTPISPPLRDSHPQAMQWNSTAFHQALQDPRVRGLYFPAGGSSSGTVNPAPNI
ASHISSISARTGDPVTNADLGYGPATPAAPAAGYTPATPAAPAEAAPAGKATTEEQ
KLIEKINAGFKAALAAAAGVQPADKYR

PreSF4 (SEQ ID No. 24):

MGKATTEEQKLIEDVNASFRAAMATTANVPPADKGKATTEEQKLIEDVNASFRAAM
ATTANVPPADKGGWSSKPRKGMGTNLSVPNPLGFFPDHQLDPAFGANSNNPDWDFN
PIKDHWPAANQVGVGAFGPGLTPPHGGILGWSPQAQGILTTVSTIPPPASTNRQSG
RQPTPISPPLRDSHPQAMQWNSTAFHQALQDPRVRGLYFPAGGSSSGTVNPAPNIA
SHISSISARTGDPVTNGKATTEEQKLIEDVNASFRAAMATTANVPPADKGKATTEE
QKLIEDVNASFRAAMATTANVPPADK

Furthermore, rabbit antibodies specific for the registered hepatitis B vaccine ENGERIX-B were obtained by immunizing New Zealand white rabbits (n=2) three-times with commercially available ready-to-use pre-filled syringes at an interval of one month.

Serum samples were obtained before immunization and approximately four weeks after the third immunization and stored at -20°C until analysis.

Immunization with PreS vaccine mixture showed induction of IgG antibodies with specificity for sequential PreS epitopes. Fig. 2 shows a comparison of the IgG antibody responses towards PreS and synthetic PreS-derived peptides induced in rabbits with CFA-formulated PreS or aluminium hydroxide-adsorbed PreS vaccine mixture (Fig. 2B). Rabbit antibodies induced with CFA-formulated PreS recognized PreS and each of the PreS-derived peptides except of P7 (Fig. 2A). Aluminium-hydroxide adsorbed PreS vaccine mixture in a 20µg dose induced PreS-specific IgG antibodies and IgG antibodies directed mainly to the N-terminal peptides P1, P2, peptide P6 and towards the C-terminal peptide P8 (Fig. 2B). No PreS or peptide-specific IgG responses were found in rabbits before immunization (Fig. 2, left bars).

### Example 3: Assessment of PreS- and PreS peptide-specific humoral immune responses

Serum samples were obtained from patients who have received three injections of Al(OH)₃-adsorbed PreS vaccine mixture (i.e., mixes of 10, 20 or 40 µg of each PreS vaccine mixture component or placebo, i.e., Al(OH)₃). Sera were collected before and four weeks after the third immunization and stored at -20°C until use. A second set of serum samples was obtained from patients who were treated over a period of two years with seven subcutaneous injections of Al(OH)₃-adsorbed PreS vaccine mixture (i.e., mixes of 20 or 40 µg of each PreS vaccine mixture component or Al(OH)₃ as placebo). In addition, serum samples were obtained from patients suffering from hepatitis B infection which was diagnosed based on clinical data, liver function testing and HBV serum markers.

All sera analyzed, were screened for serological markers for HBV (i.e., hepatitis B surface antigen [HBsAg]; antibodies to the hepatitis B surface antigen [anti-HBs] as well as antibodies to the hepatitis B core antigen [anti-HBc].

ELISA plates (NUNC MaxiSorp®, Denmark) were coated with the antigens (recombinant PreS, synthetic PreS-overlapping peptides: P1-P8) or human serum albumin (negative control) (Behring, USA). Incubation was performed with rabbit sera in a dilution of 1:10,000 (CFA) or 1:500 (PreS vaccine mixture-20/ PreS vaccine mixture-40), with mouse sera in a dilution of 1:1,000 and with human sera diluted differently for the isotypes and IgG subclasses. For the detection of human total IgG, sera were diluted 1:100, for IgA, IgG1, IgG2, IgG3, IgG4 as well as IgM, sera were diluted 1: 20 and for detection of IgE antibodies sera were diluted 1:10.

Rabbit IgG was detected with donkey anti-rabbit horse radish peroxidase-conjugated IgG antibodies, diluted 1:2,500 (GE Healthcare, Buckinghamshire, Great Britain). Bound mouse IgG1 was detected with monoclonal rat anti-mouse IgG1 (BD Pharmingen, USA) diluted 1:1,000, followed by horse radish peroxidase-conjugated goat anti-rat IgG antibodies (Amersham Bioscience, Sweden) diluted 1:2,500.

Human IgG was detected with rabbit anti-human IgG Fc-specific antibody (Jackson-Dianova, Germany) diluted 1:10,000, followed by peroxidase-linked donkey anti-rabbit IgG (GE Healthcare) at a dilution of 1:2,500. Human IgA, IgG subclasses IgG1, IgG2 and IgG4 as well as human IgM were detected with purified mouse anti-human IgA1/IgA2, IgG1, IgG2, IgG4 and IgM (BD Pharmingen) antibodies, diluted 1:1,000 respectively, followed by peroxidase-linked sheep anti mouse IgG (GE Healthcare) at a dilution of 1:2,500. Monoclonal anti-human IgG3 (Sigma Aldrich, USA) was diluted 1:5,000. Human IgE was detected with goat anti-human horse radish peroxidase-conjugated IgE antibodies (KPL, USA).

### Example 4: PreS-specific antibody responses of PreS vaccine mixture immunized subjects are not influenced by prior hepatitis B immunity

Serum samples from human subjects who received immunotherapy with PreS vaccine mixture or with placebo were tested for IgG reactivity to PreS and synthetic PreS peptides (Figs. 3a to 3c). These patients (n=30) had been screened for hepatitis B-specific serum markers (HBsAg, anti-HBs and anti-HBc antibodies) before treatment and found to be negative for HBsAg and anti-HBc antibodies. Due to previous vaccination with a hepatitis B vaccine, twenty-two of the subjects contained anti-HBs antibodies (Figs. 3a to 3c). It was found that each of the patients who received immunotherapy with PreS vaccine mixture, regardless if they had been HB-vaccinated before or not, but not placebo-treated patients developed robust PreS-specific IgG responses when sera were tested after the third (V8; three months after first injection) as well as after the seventh injection (V15; 15 months after first injection) (Figs. 3a to 3c). The PreS-specific IgG responses increased significantly from baseline before immunotherapy (i.e., V5 versus V8) and further increased significantly between V8 and V15 (i.e., after the seventh injection) (Figs. 3a to 3c). The PreS-specific IgG responses in these patients were directed mainly towards the N-terminal peptides P1, P2 and P3 and again P1- and P2-specific IgG responses showed significant increases from baseline V5 to V8 and from V8 to V15 (Figs. 3a to 3c). Also increases of IgG responses against the other PreS-derived peptides P4, P5, P6, P7 and P8 were found in sera from patients who received immunotherapy with PreS vaccine mixture but not in placebo-treated patients (Figs. 3a to 3c).

### Example 5: PreS-specific antibody responses of PreS vaccine mixture immunized subjects are directed against neutralizing epitopes and differ from those of hepatitis B-infected individuals

Fig. 4 shows a comparison of the PreS-specific isotype and IgG subclass responses of patients after immunotherapy with PreS vaccine mixture or placebo with that of hepatitis B-infected individuals. Immunotherapy with both doses of PreS vaccine mixture induced a robust PreS-specific IgG response in each of the treated patients which was significantly higher than the IgG response in hepatitis B-infected individuals (Fig. 4). No relevant PreS-specific IgA, IgE or IgM responses were detected in sera from patients who were treated with PreS vaccine mixture or placebo as well as in hepatitis B-infected individuals (Fig. 4). The PreS-specific IgG subclass response was different between PreS vaccine mixture-treated subjects and hepatitis B-infected individuals. PreS vaccine mixture-treated subjects showed a preferential IgG1 and IgG4 subclass response to PreS whereas hepatitis B-infected individuals mounted some IgG1 and IgG2 responses towards PreS (Fig. 4).

Also striking differences regarding the epitope specificity of PreS-specific antibodies in PreS vaccine mixture-treated patients versus hepatitis B-infected individuals were found (Fig. 5). PreS vaccine mixture-immunized patients but not hepatitis B-infected individuals showed strong IgG responses towards P1 and P3 (Fig. 5). This finding is surprising because the region defined by P1 corresponds to a motif within PreS1 (see also Fig. 1) that has been reported to contain the essential residues for inhibition of hepatitis B-infections. Furthermore, P7 was recognized only by PreS vaccine mixture-treated subjects but not by hepatitis B-infected individuals whereas IgG responses towards P2 and P6 were also found in hepatitis B-infected individuals (Fig. 5).

### Example 6: Assessment of T cell responses

Peripheral Blood Mononuclear Cells (PBMC) were obtained from heparinized blood samples through density gradient centrifugation using Ficoll (Amersham Biosciences, Sweden). When blood samples could be obtained, PreS-specific PBMC proliferation was determined in PreS vaccine mixture-vaccinated subjects (n=19) at V5, V8, M1 (5 months after first vaccination) and M2 (17 months after first vaccination) by [3H]-thymidine incorporation.

For certain PreS vaccine mixture-immunized patients (n=11) CD4 and CD8 T cell responses could be assessed at M2 by carboxyfluorescein succinimidyl ester (CFSE) labelling.

Fluorescent dye-labelled cells were seeded at 200,000 cells/well in Ultra culture™ serum-free medium (Lonza, Belgium) supplemented with 2 mmol/L L-glutamine (Sigma Aldrich, USA), 50mmol/L β-mercaptoethanol (Sigma Aldrich), and 0.02 mg of gentamicin per milliliter (Sigma Aldrich), in a total volume of 200µl in 96 well microplates with U shaped bottom (Thermo Fisher, USA). Cells were either left unstimulated (negative control) or were stimulated with Dynabeads® Human T-Activator CD3/CD28 (3µg/well (Invitrogen, USA)) as positive control or with PreS (0.15µg/well), equimolar quantities of PreS-overlapping peptides (0.03µg/well) or with a mixture of the PreS-derived overlapping peptides containing 0.03µg/well of each peptide and cultured at 37 °C in 5% CO₂ for 7 days before antibody staining and FACS analysis was conducted.

For flow cytometry the following reagents were used: PerCP/Cy5.5 anti-human CD3 antibody (Clone HIT3a), Brilliant Violet 421™ anti-human CD4 antibody (Clone RPA-T4), APC anti-human CD8a antibody (Clone HIT8a), as well as isotype controls, i.e., PerCP/Cy5.5 mouse IgG2a, Brilliant Violet 421™ mouse IgG1, APC mouse IgG1 (BioLegend, USA) and Fixable Viability Dye eFluor® 780 (eBioscience, USA).

Flow Cytometry was performed on a BD FACS Canto II (Becton, Dickinson and Company, USA). Twenty thousand events were acquired per sample and analysis was performed via FlowJo Software, Version 10. Lymphocytes were gated according to morphological criteria on a forward and sideward scatter dot blot, dead cells were excluded by staining of viability dye and gating was focused on CD3CD4 and CD3CD8-positive T cells. Those cells that proliferated in response to antigen stimulation were identified by their reduction in CFSE fluorescence intensity. Results represent means of triplicate cultures and 235 median percentages stimulation of CD3+CD4+ and CD3+CD8+ above background are shown for the different antigens and the analysed patients.

Fig. 6 shows the development of PreS-specific T cell responses in patients who received immunotherapy with PreS vaccine mixture. A gradually increasing PreS-specific T cell response was found which was significantly higher at V8, M1 and M2 as compared to baseline at V5 (Fig. 6A). When the epitope specificity of the PreS-specific CD4 cell responses was analyzed by CFSE staining we found that P1, P2, P5 and P6 induced the strongest CD4 cell proliferation but CD4 responses towards P3, P4 and P7 were also found (Fig. 6B). Interestingly, the peptides and the peptide mix induced stronger CD4 cell proliferation than the PreS protein (Fig. 6B). Albeit at low frequency, some PreS and PreS peptide-specific CD8 cell response was detected which was mainly directed towards P2, P3, P6 and P8 and complete PreS (Fig. 6B).

### Example 7: Hepatitis B virus neutralization assays

The HBV inoculum for infection was prepared from supernatants of HepAd38 cells using a heparin column (GE Healthcare, Great Britain) to isolate viral particles. HepG2-hNTCP cells20 were seeded at a density of 3x10⁵ cells /well in a 24 well plate. At day two after seeding, the infection medium (DMEM, Invitrogen, USA) was supplemented with 2.5% DMSO (Merck, Germany) and at day three cells were infected with HBV. For the neutralization of HBV particles, patients' sera (10µl) were preincubated with the HBV inoculum (6.9 x 10⁷ genome equivalents (GE) / well) for 30 minutes at 37°C, followed by co-incubation of cells with the patients' sera and virus in presence of 4% polyethylene glycol 800 (Sigma Aldrich, USA) for 16 hours at 37°C. The neutralizing monoclonal antibody Ma18/721 was used as positive control.

After 16 hours of inoculation, cells were washed extensively with PBS and fresh differentiation medium, supplemented with 2.5% DMSO (Invitrogen) was added. Additional medium changes were performed at day three and day five post infection.

Quantification of HBV infection was conducted by the measurement of secreted hepatitis B e antigen (HBeAg) in the supernatant from cells at day five to seven after infection. HBeAg was determined by ADVIA Centaur XPT automated chemiluminescence system (Siemens, Germany). Samples were considered as positive at a signal above 1 Index.

The expression of HBV core protein was detected by specific immunofluorescence. The supernatant was removed and the cells were washed with PBS prior to the fixation with 4% paraformaldehyde (Sigma Aldrich) for 30 minutes at room temperature (RT). Next, cells were washed with PBS followed by the permeabilization with 0.25% Triton X 100 (AppliChem GmbH, Germany) in PBS for 30 minutes at RT. Then, cells were incubated overnight at 4°C with the primary antibody (anti-HBV core, rabbit polyclonal AK, DAKO Deutschland GmbH, Hamburg, Germany) diluted in 2% w/v BSA, PBS. On the next day, cells were washed with PBS and finally incubated with the secondary antibody (goat α rabbit Alexa 488; Invitrogen, Carlsbad, CA) and 4', 6-Diamidin-2-phenylindo / Hoechst 33342 (Roche Applied Science, Germany) in the dark. For the detection of HBV core protein, the secondary antibody was incubated for 2 hours at RT, protected from light. Cells were examined under fluorescence microscope using 480nm for Alexa-488-labeled secondary antibodies (Invitrogen, Carlsbad, CA) and 360nm for the nuclear staining.

In the first type of assay the expression of hepatitis B core antigen (HBcAg) after infection of cells is detected by specific immunofluorescence. No HBcAg has been detected in uninfected cells but in infected and untreated cells and that expression can be prevented by pre-incubation of virus with the neutralizing monoclonal antibody Ma18/721 which is directed against the PreS1 domain of the large hepatitis B surface protein. Likewise it was found that pre-incubation of hepatitis B virus with rabbit antibodies induced by the commercial vaccine Engerix-B or with rabbit anti-PreS vaccine mixture (20µg dose) antibodies inhibited infection of HepG2-hNTCP cells. A similar set of experiments was performed with sera from PreS vaccine mixture- or placebo-treated patients. Sera obtained from a patient before and after immunization with placebo did not inhibit infection of HepG2-hNTCP cells whereas sera obtained from a patient after immunization with 20 µg or from a patient after immunization with 40 µg inhibited infection of HepG2-hNTCP cells.

In addition to the staining of the HBcAg an assay based on the measurement of secreted hepatitis B e antigen (HBeAg) by HepG2-hNTCP cells was used seven days post infection with HBV as another surrogate marker to quantify the inhibition of HBV infection. It was found that sera from PreS vaccine mixture-treated inhibited HBV infection between 50-99% (Fig. 7A). No relevant difference was found depending on the dose and number of PreS vaccine mixture injections because a similar inhibition was observed for sera from patients who had received three injections (Fig. 7A) as well as for sera from patients who had received seven injections (Fig. 7A, black). Also, there was no obvious difference regarding the degree of inhibition between patients who either received the 20 µg or 40 µg dose of PreS vaccine mixture (Fig. 7A). No inhibition was observed for serum from a placebo treated patient and a more than 90% inhibition was observed for the monoclonal antibody Ma 18/7 (Fig. 7A). Rabbit anti-Engerix-B and rabbit anti-PreS vaccine mixture antibodies caused a more than 99% inhibition of HBV infection (Fig. 7B).

### Example 8:

Recombinant PreS and human serum albumin (Behring, USA) as negative control were coated onto Nunc Maxisorb microplates (Thermo-Fisher Scientific, USA) at a concentration of 2µg/ml in 100mM sodium phosphate buffer, pH 9.6 overnight at 4°C. Wash buffer was comprised of PBS, 0.05% v/v Tween20 (PBS/T) and the blocking procedures were performed with 2% w/v BSA, PBS/T for 2 hours at 37°C. All subsequent serum and reagent dilutions were done in 0.5% w/v BSA, PBS/T.

To determine humoral immune responses of rabbits, which have undergone complete immunization, either with recombinant PreS or PreS-fusion-proteins, as emulsion in Complete Freund's Adjuvant (CFA), sera were used in different dilutions (4°C, overnight) and bound total rabbit IgG was detected using donkey anti-rabbit horse radish peroxidase-conjugated IgG antibodies diluted 1:2.000 (GE Healthcare, Great Britain). The color reaction was induced by ABTS [2, 2'-azino-bis (3-ethylbenzothiazoline-6-sulphonic acid] and absorbance detection, corresponding to the levels of antigen-specific antibodies was performed at 405nm and 490nm using a microplate reader (Molecular Devices, USA). All determinations were performed in triplicates.

It surprisingly turned out that only a fusion protein comprising one or more peptides having the amino acid sequences SEQ ID No. 1, SEQ ID No. 2, SEQ ID No. 3 and/or SEQ ID No. 4 and PreS (PreS-F3) were able to induce the formation of PreS specific IgG to a much higher extend compared to PreS alone or other fusion proteins comprising also PreS fused to different peptides (PreS-F1, PreS-F2, PreS-F4) as depicted in Fig. 8.

## Claims

1. Fusion protein for use in the treatment and/or prevention of a hepatitis B virus infection comprising at least one hepatitis B PreS polypeptide or fragment thereof fused to at least one peptide consisting of an amino acid sequence having at least 80% identity to a sequence selected from the group consisting of SEQ ID No. 1, SEQ ID No. 2, SEQ ID No. 3 and SEQ ID No. 4.

2. Fusion protein for the use according to claim 1, wherein the amino acid sequence of the PreS polypeptide is at least 80% identical to SEQ ID No. 5, SEQ ID No. 7, SEQ ID No. 8, SEQ ID No. 9, SEQ ID No. 10, SEQ ID No. 11, SEQ ID No. 12 or SEQ ID No. 13.

3. Fusion protein for the use according to claim 1 or 2, wherein the at least one peptide is fused to the N- and/or C-terminus of the PreS polypeptide.

4. Fusion protein for the use according to any one of claims 1 to 3, wherein the fusion protein comprises an amino acid sequence which is at least 80% identical to SEQ ID No. 6.

5. Fusion protein for the use according to any one of claims 1 to 4, wherein the hepatitis B virus infection is caused by a hepatitis B virus genotype A, B, C, D, E, F, G, H or a subtype thereof.

6. Fusion protein for the use according to any one of claims 1 to 5, wherein the fusion protein is administered to an individual at least once in an amount of 0.01 µg/kg body weight to 5 mg/kg body weight, preferably 0.1 µg/kg body weight to 2 mg/kg body weight.

7. Fusion protein for the use according to any one of claims 1 to 6, wherein the fusion protein is administered together with at least one adjuvant and/or pharmaceutical acceptable excipient.
